# EUROPÄISCHE PATENTSCHRIFT

(11) **EP 3 236 926 B1**
(45) Veröffentlichungstag und Bekanntmachung des Hinweises auf die Patenterteilung: **26.02.2020**
(21) Anmeldenummer: 15790102.6
(22) Anmeldetag: 02.11.2015
(51) Int. Cl.: A61K 8/49, A61K 8/34, A61K 8/04, A61Q 5/06

(54) **SCHAUMFÖRMIGE DIREKTZIEHENDE FÄRBEMITTEL**
DIRECT DYEING AGENTS IN FOAM FORM
COLORANT DIRECT EN FORME DE MOUSSE

(30) Priorität: 22.12.2014 DE 102014226752
(43) Veröffentlichungstag der Anmeldung: 01.11.2017
(73) Patentinhaber: Henkel AG & Co. KGaA, 40589 Düsseldorf (DE)
(72) Erfinder: GOUTSIS, Konstantin, 41363 Jüchen (DE); WESER, Gabriele, 41472 Neuss (DE)
(86) Internationale Anmeldenummer: PCT/EP2015/075429
(87) Internationale Veröffentlichungsnummer: WO 2016/102106

(56) Entgegenhaltungen:
- WO-A1-2015/086283
- DE-A1- 19 653 496
- DE-A1-102011 089 223
- JP-B2- 3 577 862
- US-A- 6 099 828

## Beschreibung

Der Gegenstand der vorliegenden Anmeldung ist ein Mittel zum Färben von keratinischen Fasern, insbesondere menschlichen Haaren, welches auf einen sauren pH-Wert eingestellt ist, mindestens einen direktziehenden Säurefarbstoff und eine spezielle Mischung aus zwei organischen Lösemitteln enthalt und welches geeignet sind, aus spezielle Applikationsvorrichtungen in Form eines stabilen Schaums ausgetragen zu werden. Ein weiterer Gegenstand der vorliegenden Anmeldung ist ein Verfahren zum Färben von keratinischen Fasern, in dem ein entsprechendes Mittel auf keratinische Fasern appliziert wird.

Die Veränderung von Form und Farbe von keratinischen Fasern, insbesondere von Haaren, stellt einen wichtigen Bereich der modernen Kosmetik dar. Hierdurch kann das Erscheinungsbild der Haare sowohl aktuellen Modeströmungen als auch den individuellen Wünschen der einzelnen Person angepasst werden. Die Färbung, insbesondere die Abdeckung von ergrauten Haaren, wird von vielen Menschen angestrebt.

Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden üblicherweise Oxidationsfärbemittel verwendet Solche Färbemittel enthalten üblicherweise Oxidationsfarbstoffvorprodukte, sogenannte Entwickler- und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch hervorragende, lang anhaltende Färbeergebnisse aus, sind jedoch auch mit einem gewissen Ausmaß an Haarschädigung verbunden.

Durch den Einsatz von direktziehenden Farbstoffen kann die Haarfarbe temporär verändert werden. Hierbei diffundieren bereits fertig ausgebildete Farbstoffe aus dem Färbemittel in die Haarfaser hinein. Zur Verbesserung der Farbstoffdiffusion ist es üblich, der Färbemittelrezeptur ein oder mehrere organische Lösemittel, ausgewählt aus niederen Alkylencarbonaten, wie Propylencarbonat, aromatischen Alkoholen, insbesondere Benzylalkohol, Phenoxyethanol oder Benzyloxyethanol, und N-Alkylpyrrolidonen, insbesondere N-Methylpyrrolidon, zuzusetzen.

Im Vergleich zur oxidativen Haarfärbung weisen die mit direktziehenden Farbstoffen erhaltenen Färbungen eine geringere Haltbarkeit und schnellere Auswaschbarkeit auf. Auch die Grauabdeckung, die mit direktziehenden Farbstoffen erhalten werden kann, ist in der Regel verbesserungswürdig. Von Vorteil ist jedoch die geringere Haarschädigung der Färbung mit direktziehenden Farbstoffen. Um die Haarschädigung so gering wie möglich zu halten, ist der Einsatz von direktziehenden Farbstoffen daher die Färbemethode der Wahl.

Direktziehende Färbemittel werden häufig als Shampoo-Rezeptur formuliert und vermarktet. Darüber hinaus gab es häufig Versuche, andere Konfektionierungsformen zu entwickeln. So wurde vorgeschlagen, dünnflüssigere Färbemittel als Schaum zu applizieren. Bei der Schaumapplikation ist insbesondere der Einsatz von Aerosolschäumen verbreitet.

Ein Problem bei der Schaumapplikation ist die Stabilisierung der Schäume. Die Beschaffenheit von Schäumen ist dann als ideal zu bezeichnen, wenn bei der Produktabgabe ein fester, stabiler Schaum entsteht, der ein geschmeidiges Gefühl hinterlässt und auf dem Haar nur langsam bricht. Häufig ist aber zu beobachten, dass die ausgebrachten Schäume wenig stabil sind und schnell wieder in sich zusammenfallen, so dass eine dünnflüssige, tropfende Lösung zurückbleibt. Andererseits ist es auch wesentlich, dass der Schaum trotzdem die Haare gut benetzt, so dass ein guter Farbauftrag stattfinden kann. Die Schaumstabilität wird insbesondere durch die Anwesenheit größerer Mengen an organischen Lösemitteln negativ beeinflusst.

Aus der Offenlegungsschrift JP61210023A sind bereits schaumförmige Haarfarbemittel zur direktziehenden Haarfärbung bekannt, die als besonders geeignete Lösemittel 5 - 65 Gew.-% organische Lösemittel, darunter niedere Alkylencarbonate und aromatische Alkohole, offenbaren. Aus der Schrift DE19653496A1 sind bereits schaumförmige Haarfärbemittel zur direktziehenden Haarfärbung bekannt, die als besonders geeignete Lösemittel aromatische Alkohole und N-Alkylpyrrolidone offenbaren.

Die hieraus produzierten Schäume waren allerdings in ihren Schaumeigenschaften, insbesondere in Bezug auf das Brechen des Schaums beim Verteilen auf dem Haar, weiter verbesserungswürdig, um eine optimale Verteilbarkeit des Färbemittels und einen optimalen Farbaufzug auf das Haar zu gewährleisten.

Der vorliegenden Erfindung lag die Aufgabe zugrunde, ein Mittel zum direktziehenden Färben von keratinischen Fasern bereit zu stellen, das aus einem Spender als Schaum ausgetragen und danach als Schaum auf das Haar appliziert wird und eine Färbung mit hoher Waschechtheit und guter Grauabdeckung ermöglicht.

US 6099828A und JP 3577862B2 offenbaren direktziehende Färbemittel mit Säurefarbstoff und saurem pH. die neben Propylencarbonat ausgewählte N-Alkylpyrrolidone und Ethanol enthalten.

Es hat sich nun herausgestellt, dass sich keratinische Fasern intensiv mit einem Mittel färben lassen, das auf einen sauren pH-Wert eingestellt ist und mindestens einen direktziehenden Säurefarbstoff sowie, bezogen auf das Gewicht des Mittels, 6 bis 12 Gew.-% organisches Lösemittel, enthält, das zu 80 - 100 Gew.-% aus Propylencarbonat und zu 0 - 20 Gew.-% aus Benzylalkohol besteht.

Bei Anwendung dieses Mittels als Schaum ergibt sich eine optimale Schaumqualität, wobei der Schaum ausreichend stabil ist, um gleichmäßig im Haar verteilt werden zu können, anschließend aber auch ausreichend schnell bricht, um so einen optimalen Farbaufzug zu gewährleisten.

Ein erster Gegenstand der vorliegenden Erfindung ist ein wasserhaltiges kosmetisches Mittel zum Färben von keratinischen Fasern, welches
- einen pH-Wert im Bereich von 1,0 bis 5,5 besitzt und
- mindestens einen direktziehenden Säurefarbstoff und
- bezogen auf das Gewicht des Mittels, 6 bis 12 Gew.-% organisches Lösemittel, das zu 80 - 100 Gew.-% aus Propylencarbonat und zu 0 - 20 Gew.-% aus Benzylalkohol besteht, und mindestens ein nichtionisches ethoxyliertes Tensid in einer Gesamtmenge von 0,1 - 1 Gew.-% enthält,
wobei der Gesamtgehalt an organischem Lösemittel 6 bis 12 Gew.-%. bezogen auf das Gewicht des Mittels, beträgt.

Unter keratinischen Fasern, keratinhaltigen Fasern oder Keratinfasern sind Pelze, Wolle, Federn und insbesondere menschliche Haare zu verstehen. Obwohl die erfindungsgemäßen Mittel in erster Linie zum Färben von Keratinfasern geeignet sind, steht prinzipiell einer Verwendung auch auf anderen Gebieten nichts entgegen.

Bei dem erfindungsgemäßen Mittel handelt es sich um ein wasserhaltiges kosmetisches Mittel.

Bevorzugte Mittel enthalten, jeweils bezogen auf ihr Gewicht, 60 - 90 Gew.-% Wasser, bevorzugt 70 - 87 Gew.-% Wasser.

Als ersten wesentlichen Inhaltsstoff enthält das erfindungsgemäße Mittel mindestens einen direktziehenden Säurefarbstoff.

Direktziehende Farbstoffe können anhand ihrer Ladung in kationische Farbstoffe (basische Farbstoffe), nichtionische Farbstoffe und anionische Farbstoffe (auch Säurefarbstoffe genannt) unterteilt werden.

Unter Säurefarbstoffen werden direktziehende Farbstoffe verstanden, die mindestens eine Carbonsäuregruppierung (-COOH) und/oder eine Sulfonsäuregruppierung (-SO₃H) besitzen. In Abhängigkeit vom pH-Wert liegen die protonierten Formen (-COOH, -SO₃H) der Carbonsäure- bzw. Sulfonsäuregruppierungen mit ihren deprotonierten Formen (-COO⁻, -SO₃⁻vor) im Gleichgewicht vor. Mit abnehmendem pH-Wert steigt der Anteil der protonierten Formen. Werden direktziehende Farbstoffe in Form ihrer Salze eingesetzt, so liegen die Carbonsäuregruppen bzw. Sulfonsäuregruppen in deprotonierter Form vor und sind zur Einhaltung der Elektroneutralität mit entsprechenden stöchiometrischen Äquivalente an Kationen (wie beispielsweise Na-Kation oder K-Kationen) neutralisiert.

Ein wesentliches Merkmal der Säurefarbstoffe ist ihr Vermögen, anionische Ladungen auszubilden, wobei die hierfür verantwortlichen Carbonsäure- bzw. Sulfonsäuregruppen an verschiedene chromophore Systeme geknüpft sein können. Geeignete chromophore Systeme finden sich beispielsweise in den Strukturen von Azofarbstoffen, Triarylmethanfarbstoffen, Anthrachinonfarbstoffen, Xanthen-Farbstoffen bzw. Rhodamin-Farbstoffen und/oder Oxazinfarbstoffen.

Als geeignete Säurefarbstoffe können beispielsweise eine oder mehrere Verbindungen aus der folgenden Gruppe ausgewählt werden: Acid Yellow 1 (D&C Yellow 7, Citronin A, Ext. D&C Yellow No. 7, Japan Yellow 403, CI 10316, COLIPA n° B001), Acid Yellow 3 (COLIPA n° : C 54, D&C Yellow N° 10, Quinoline Yellow, E104, Food Yellow 13), Acid Yellow 9 (CI 13015), Acid Yellow 17 (CI 18965), Acid Yellow 23 (COLIPA n° C 29, Covacap Jaune W 1100 (LCW), Sicovit Tartrazine 85 E 102 (BASF), Tartrazine, Food Yellow 4, Japan Yellow 4, FD&C Yellow No. 5), Acid Yellow 36 (CI 13065), Acid Yellow 121 (CI 18690), Acid Orange 6 (CI 14270), Acid Orange 7 (2-Naphthol orange, Orange II, CI 15510, D&C Orange 4, COLIPA n° C015), Acid Orange 10 (C.I. 16230; Orange G sodium salt), Acid Orange 11 (CI 45370), Acid Orange 15 (CI 50120), Acid Orange 20 (CI 14600), Acid Orange 24 (BROWN 1;CI 20170;KATSU201;nosodiumsalt;Brown No.201;RESORCIN BROWN;ACID ORANGE 24;Japan Brown 201;D & C Brown No.1), Acid Red 14 (C.I. 14720), Acid Red 18 (E124, Red 18; CI 16255), Acid Red 27 (E 123, CI 16185, C-Rot46, Echtrot D, FD&C Red Nr.2, Food Red 9, Naphtholrot S), Acid Red 33 (Red 33, Fuchsia Red, D&C Red 33, CI 17200), Acid Red 35 (CI C.I.18065), Acid Red 51 (CI 45430, Pyrosin B, Tetraiodfluorescein, Eosin J, lodeosin), Acid Red 52 (CI 45100, Food Red 106, Solar Rhodamine B, Acid Rhodamine B, Red n° 106 Pontacyl Brilliant Pink), Acid Red 73 (CI CI 27290), Acid Red 87 (Eosin, CI 45380), Acid Red 95 (CI 45425, Erythtosine, Simacid Erythrosine Y), Acid Red 184 (CI 15685), Acid Red 195, Acid Violet 43 (Jarocol Violet 43, Ext. D&C Violet n° 2, C.I. 60730, COLIPA n° C063), Acid Violet 49 (CI 42640), Acid Violet 50 (CI 50325), Acid Blue 1 (Patent Blue, CI 42045), Acid Blue 3 (Patent Blau V, CI 42051), Acid Blue 7 (CI 42080), Acid Blue 104 (CI 42735), Acid Blue 9 (E 133, Patentblau AE, Amidoblau AE, Erioglaucin A, CI 42090, C.I. Food Blue 2), Acid Blue 62 (CI 62045), Acid Blue 74 (E 132, CI 73015), Acid Blue 80 (CI 61585), Acid Green 3 (CI 42085, Foodgreen1), Acid Green 5 (CI 42095), Acid Green 9 (C.1.42100), Acid Green 22 (C.I.42170), Acid Green 25 (CI 61570, Japan Green 201, D&C Green No, 5), Acid Green 50 (Brillantsäuregrün BS, C.I. 44090, Acid Brilliant Green BS, E 142), Acid Black 1 (Black n° 401, Naphthalene Black 10B, Amido Black 10B, CI 20 470, COLIPA n° B15), Acid Black 52 (CI 15711), Food Yellow 8 (CI 14270), Food Blue 5, D&C Yellow 8, D&C Green 5, D&C Orange 10, D&C Orange 11, D&C Red 21, D&C Red 27, D&C Red 33, D&C Violet 2 und/oder D&C Brown 1.

Ein ganz besonders bevorzugtes erfindungsgemäßes Mittel ist daher dadurch gekennzeichnet, dass es mindestens einen direktziehenden Säurefarbstoff aus der Gruppe Acid Yellow 1, Acid Yellow 3, Acid Yellow 9, Acid Yellow 17, Acid Yellow 23, Acid Yellow 36, Acid Yellow 121, Acid Orange 6, Acid Orange 7, Acid Orange 10, Acid Orange 11, Acid Orange 15, Acid Orange 20, Acid Orange 24, Acid Red 14, Acid Red 18, Acid Red 27, Acid Red 33, Acid Red 35, Acid Red 51, Acid Red 52, Acid Red 73, Acid Red 87, Acid Red 95, Acid Red 184, Acid Red 195, Acid Violet 43, Acid Violet 49, Acid Violet 50, Acid Blue 1, Acid Blue 3, Acid Blue 7, Acid Blue 104, Acid Blue 9, Acid Blue 62, Acid Blue 74, Acid Blue 80, Acid Green 3, Acid Green 5, Acid Green 9, Acid Green 22, Acid Green 25, Acid Green 50, Acid Black 1, Acid Black 52, Food Yellow 8, Food Blue 5, D&C Yellow 7, D&C Yellow 8, D&C Orange 4, D&C Green 5, D&C Orange 10, D&C Orange 11, D&C Red 21, D&C Red 27, D&C Red 33, D&C Violet 2 und/oder D&C Brown 1 enthält.

Es hat sich gezeigt, dass bestimmte Säurefarbstoffe, insbesondere bestimmte Kombinationen von Säurefarbstoffen, besonders gut zur Anwendung auf geschädigtem Haar geeignet sind, da diese besonders gut auf die geschädigten Keratinfasern aufziehen und dort zu außergewöhnlich intensiven Farbergebnissen führen. Besonders gut ziehen die folgenden Farbstoffkombinationen auf geschädigte Keratinfasern auf: Acid Yellow 1/ Acid Orange 7; Acid Yellow 3/ Acid Orange 7; Acid Yellow 9/ Acid Orange 7; Acid Yellow 17/ Acid Orange 7; Acid Yellow 23/ Acid Orange 7; Acid Yellow 36/ Acid Orange 7; Acid Yellow 121/ Acid Orange 7; Acid Red 14/ Acid Orange 7; Acid Red 18/ Acid Orange 7; Acid Red 27/ Acid Orange 7; Acid Red 33/ Acid Orange 7; Acid Red 35/ Acid Orange 7; Acid Red 51/ Acid Orange 7; Acid Red 52/ Acid Orange 7; Acid Red 73/ Acid Orange 7; Acid Red 87/ Acid Orange 7; Acid Red 95/ Acid Orange 7; Acid Red 184/ Acid Orange 7; Acid Red 195/ Acid Orange 7; Acid Violet 43/ Acid Orange 7; Acid Violet 49/ Acid Orange 7 und/oder Acid Violet 50/ Acid Orange 7.

Ein weiterhin ganz besonders bevorzugtes erfindungsgemäßes Mittel ist daher dadurch gekennzeichnet, dass es mindestens eine der folgenden Kombinationen aus direktziehenden Säurefarbstoffen enthält: Acid Yellow 1/ Acid Orange 7; Acid Yellow 3/ Acid Orange 7; Acid Yellow 9/ Acid Orange 7; Acid Yellow 17/ Acid Orange 7; Acid Yellow 23/ Acid Orange 7; Acid Yellow 36/ Acid Orange 7; Acid Yellow 121/ Acid Orange 7; Acid Red 14/ Acid Orange 7; Acid Red 18/ Acid Orange 7; Acid Red 27/ Acid Orange 7; Acid Red 33/ Acid Orange 7; Acid Red 35/ Acid Orange 7; Acid Red 51/ Acid Orange 7; Acid Red 52/ Acid Orange 7; Acid Red 73/ Acid Orange 7; Acid Red 87/ Acid Orange 7; Acid Red 95/ Acid Orange 7; Acid Red 184/ Acid Orange 7; Acid Red 195/ Acid Orange 7; Acid Violet 43/ Acid Orange 7; Acid Violet 49/ Acid Orange 7 oder Acid Violet 50/ Acid Orange 7.

Die direktziehenden Säurefarbstoffen sind bevorzugt in einer Gesamtmenge von 0,01 bis 5,5 Gew.-%, bevorzugt von 0,08 bis 4,7 Gew.-%, weiter bevorzugt von 0,2 bis 3,4 Gew.-% und besonders bevorzugt von 0,3 bis 1,8 Gew.-%, jeweils bezogen auf des Gewicht des Mittels, enthalten. Vor allem, wenn der Gesamtgehalt 0,3 bis 1,8 Gew.-% beträgt, bezogen auf das Gewicht des Mittels, können sehr intensive Färbungen erzielt werden, ohne dass die Mittel eine zu starke Hautanfärbung aufweisen.

Ein ganz besonders bevorzugtes erfindungsgemäßes Mittel ist daher dadurch gekennzeichnet, dass es ein oder mehrere direktziehende Säurefarbstoffe in einer Gesamtmenge von 0,01 bis 5,5 Gew.-%, bevorzugt von 0,08 bis 4,7 Gew.-%, weiter bevorzugt von 0,2 bis 3,4 Gew.-% und besonders bevorzugt von 0,3 bis 1,8 Gew.-% - bezogen auf des Gesamtgewicht des Mittels - enthält.

Säurefarbstoffe benötigen in der Regel ein saures Mileu, um auf die Keratinfasern aufzuziehen. Um einen ausreichenden Farbaufzug der direktziehenden Säurefarbstoffe zu gewährleisten, müssen die erfindungsgemäßen Mittel auf einen sauren pH-Wert eingestellt sein. Hierbei ziehen die direktziehenden Säurefarbstoffe umso besser auf, je saurer das Mittel eingestellt ist. Geeignet ist ein pH-Wert bis maximal 5,5, jedoch wird die Intensität der Färbung ungleich intensiver, wenn der pH-Wert der Mittel bei einem Wert bis maximal 4,7, bevorzugt bis maximal 3,9, weiter bevorzugt bis maximal 3,1 und ganz besonders bevorzugt bis maximal 2,5 liegt. Aus toxikologischen Gründen ist es nicht angezeigt, das Mittel auf pH-Werte von weniger als 1,0 einzustellen. Bevorzugt liegt der pH-Wert der Mittel nicht unterhalb von 1,5, weiter bevorzugt nicht unterhalb von 1,7, und ganz bevorzugt nicht unterhalb von 1,8, Ein weiterhin ganz besonders bevorzugtes erfindungsgemäßes Mittel ist daher dadurch gekennzeichnet, dass es einen pH-Wert von 1,5 bis 4,7, bevorzugt von 1,6 bis 3,9, weiter bevorzugt von 1,7 bis 3,1 und besonders bevorzugt von 1,8 bis 2,5 besitzt.

Zur Einstellung der sauren pH-Werte kommen prinzipiell alle Verbindungen in Frage, die zur Abgabe eines Protons (einwertige Säure) oder mehrerer Protonen (mehrwertige Säure) in der Lage sind. Als anorganische Säuren können beispielsweise Mineralsäuren wie Salzsäure, Schwefelsäure und Phosphorsäure, bevorzugt in ihrer mit Wasser verdünnten Form, verwendet werden. Auch organische Sauren können in den erfindungsgemäßen Formulierungen eingesetzt werden. Typische Vertreter für organische Säuren sind aliphatische Mono- und Dicarbonsäuren wie beispielsweise Essigsäure, Propionsäure, Oxalsäure und 1,3-Propandisäure sowie aromatische Carbonsäuren wie beispielsweise Benzoesäure. Weitere erfindungsgemäße organische Säuren sind Hydroxycarbonsäuren wie Glykolsäure, Zitronensäure, Weinsäure, Äpfelsäure und Milchsäure. Auch ungesättigte Mono- oder Dicarbonsäuren wie beispielsweise Fumarsäure oder alpha-Ketocarbonsäuren wie beispielsweise Brenztraubensäure (2-Oxopropionsäure) sind erfindungsgemäß geeignet.

Aufgrund der für kosmetische Mittel bestehenden formulierungstechnischen und gesetzlichen Anforderungen sind jedoch geruchsarme, für den Einsatz in Kosmetika zugelassene Säuren am besten geeignet zur Entwicklung von Haarbehandlungsmitteln mit guter Färbeleistung. Haarfärbemittel, die mindestens eine Säure ausgewählt aus Zitronensäure, Weinsäure, Äpfelsäure, Milchsäure, 1-Hydroxyethan-1,1-diphosphonsäure, 2,6-Dipicolinsäure und Benzoesäure enthalten, sind daher bevorzugt.

Bei den im Sinne dieser Erfindung gemessenen pH-Werten handelt es sich um pH-Werte, die bei einer Temperatur von 22 °C gemessen wurden. Zur Messung des pH-Wertes eignen sich insbesondere Glaselektroden, die beispielsweise in Form einer Einstabmesskette ausgeführt sein können.

Die Einhaltung der vorgenannten geeigneten, bevorzugten und besonders bevorzugten pH-Wert Bereiche ist zur Erzielung eines zufriedenstellenden Farbergebnisses essentiell. Darüber hinaus hat sich herausgestellt, dass es auch zur optimalen Reduzierung der Haarschädigung wichtig ist, den pH-Wert zuverlässig auf die gewünschten und bevorzugten Bereiche einzustellen. Um den eingestellten pH-Wert auch langfristig im gewünschten Bereich zu halten, ist daher insbesondere auch der Einsatz von Puffern - d.h. der Einsatz einer schwachen Säure (z.B. Essigsäure) mit einem praktisch völlig dissoziierten Salz derselben Säure (z.B. Natriumacetat) - bevorzugt. Bestimmte Puffersysteme haben hierbei besondere Eignung gezeigt.

Ein weiterhin ganz besonders bevorzugtes erfindungsgemäßes Mittel ist daher dadurch gekennzeichnet, dass es mindestens ein Gemisch aus einer Säure und dem Alkalisalz dieser Säure enthält, wobei die Säure ausgewählt ist aus der Gruppe Zitronensäure, Weinsäure, Äpfelsäure, Milchsäure und 1-Hydroxyethan-1,1-diphosphonsäure.

Unter Zitronensäure (Alternativname: 2-Hydroxypropan-1,2,3-tricarbonsäure) wird die die D-Form der Säure, die L-Form der Säure wie auch die Gemische hiervon verstanden.

Weinsäure (Alternativnamen: 2,3-Dihydroxybutandisäure, 2,3-Dihydroxybernsteinsäure) tritt in 3 stereoisomeren Formen auf: die Enantiomere L-(+)-Weinsäure und die D-(-)-Weinsäure sowie die optisch inaktive meso-Form. Erfindungsgemäß sind alle diese stereoisomeren Formen der Weinsäure und ihre Gemische.

Äpfelsäure (Alternativnamen: Hydroxybernsteinsäure, Hydroxybutandisäure) kommt in Form ihrer R-(+)-Form sowie der enantiomeren S-(-)-Form vor. Erfindungsgemäß sind jede dieser Formen sowie ihr Gemisch.

Unter Milchsäure (Alternativnamen: 2-Hydroxypropansäure, 2-Hydroxypropionsäure) wird die D-Form der Säure, die L-Form der Säure wie auch die Gemische hiervon verstanden.

Bei den vorgenannten Säuren handelt es sich um einwertige Säuren (wie z.B. Milchsäure) oder auch mehrwertige Säuren (Zitronensäure, Weinsäure, Apfelsäure). Erfindungsgemäß unter Alkalisalzen dieser Säuren die ein oder mehrwertigen (im Fall der mehrwertigen Säuren) Kalium- und Natriumsalze dieser Säuren verstanden.

Ganz besonders bevorzugt ist demnach auch ein wasserhaltiges kosmetisches Mittel zum Färben von keratinischen Fasern, welches
- einen pH-Wert im Bereich von 1,0 bis 5,5 besitzt,
- mindestens einen direktziehenden Säurefarbstoff,
- bezogen auf das Gewicht des Mittels, 6 bis 12 Gew.-% organisches Lösemittel, das zu 80 - 100

Gew.-% aus Propylencarbonat und zu 0 - 20 Gew.-% aus Benzylalkohol besteht, mindestens ein nichtionisches ethoxyliertes Tensid in einer Gesamtmenge von 0,1 - 1 Gew.-%, und
- ein Gemisch aus einer Säure und dem Alkalisalz dieser Säure enthalt, wobei die Säure ausgewählt ist aus der Gruppe Zitronensäure, Weinsäure, Äpfelsäure, Milchsäure und 1-Hydroxyethan-1,1-diphosphonsäure,
enthält,
wobei der Gesamtgehalt an organischem Lösemittel 6 bis 12 Gew.-%. bezogen auf das Gewicht des Mittels, beträgt.

Zur reproduzierbaren und zuverlässigen Stabilisierung des pH-Wertes haben sich insbesondere die Puffersysteme als geeignet erweisen, die aus einer Mischung der folgenden Säuren und ihrer Salze bestehen:
- Milchsäure und einem Natriumsalz der Milchsäure, und/oder
- Milchsäure und einem Kaliumsalz der Milchsäure,
- Zitronensäure und einem Natriumsalz der Zitronensäure,
- Zitronensäure und einem Kaliumsalz der Zitronensäure,
- Weinsäure und einem Natriumsalz der Weinsäure,
- Weinsäure und einem Kaliumsalz der Weinsäure,
- 1-Hydroxyethan-1-1-disphoshonsäure und einem Natriumsalz der 1-Hydroxyethan-1-1-disphosphonsäure,
- 1-Hydroxyethan-1-1-disphoshonsäure und einem Kaliumsalz der 1-Hydroxyethan-1-1-disphosphonsäure.

Als weiteren wesentlichen Inhaltsstoff enthalten die erfindungsgemäßen Mittel, bezogen auf ihr Gewicht, 6 bis 12 Gew.-% organisches Lösemittel, das zu 80 - 100 Gew.-% aus Propylencarbonat und zu 0 - 20 Gew.-% aus Benzylalkohol besteht.

Im Stand der Technik ist bereits bekannt hat, dass der Farbaufzug direktziehender Säurefarbstoffe auf Keratinfasern durch Einsatz eines organischen Lösungsmittels weiter verbessert werden kann. Hierbei haben sich bestimmte Lösungsmittel als besonders geeignet herausgestellt. Durch den Einsatz von Isopropanol und auch anderen aliphatischen Alkoholen, wie Ethanol konnte der Farbaufzug der Säurefarbstoffe beispielsweise nicht verbessert werden. Aromatische Alkohole aber, wie Benzylalkohol, führen zu einer signifikanten Verstärkung der Farbintensität.

Bei Benzylalkohol handelt es sich um einen aromatischen Alkohol der Formel (I).

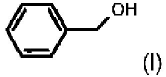

Propylencarbonat wird alternativ auch als 4-Methyl-1,3-dioxolan-2-on bezeichnet und besitzt die Struktur der Formel (II).

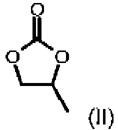

Sowohl Benzylalkohol allein als auch Propylencarbonat allein ergeben hervorragende Färbeergebnisse. Propylencarbonat ist allerdings ein deutlich teurerer Rohstoff als Benzylalkohol, daher sollte bevorzugt ein Mittel mit möglichst geringem Propylencarbonat eingesetzt werden, ohne dabei jedoch Einbußen im Färbeergebnis oder in den Anwendungseigenschaften hinzunehmen.

Es wurde beobachtet, dass die Auswahl des organischen Lösemittels zur Verbesserung des Farbaufzugs des direktziehenden Säurefarbstoffs kritisch für die Schaumstabilität ist. Es zeigte sich, dass der Gesamtgehalt an organischem Lösemittel 6 bis 12 Gew.-%, bevorzugt 10 bis 12 Gew.-%, jeweils bezogen auf das Gewicht des Mittels, betragen musste, um einen guten Farbaufzug und eine hohe Qualität des Färbeergebnisses zu erzielen. Der Einsatz von 10 Gew.-% Benzylalkohol allein allerdings beeinträchtigte die Schaumstabilität in unakzeptabler Weise.

Der Einsatz von 10 Gew.-% Propylencarbonat allein ergab sowohl hervorragende Färbeergebnisse als auch eine sehr gute Schaumstabilität. Darüber hinaus wurde untersucht, inwieweit man aus ökonomischen Gründen den Gehalt an Propylencarbonat durch Benzylalkohol ersetzen konnte. Dabei wurde gefunden, dass ein optimales Färbeergebnis verbunden mit optimalen Schaumeigenschaften durch ein Mittel erhalten wurden, dass, jeweils bezogen auf sein Gewicht, 6 - 12 Gew.-%, bevorzugt 10 - 12 Gew.-%, organisches Lösemittel enthält, das zu 80 - 100 Gew.-%, bevorzugt zu 85 - 97 Gew.-%, aus Propylencarbonat und zu 0 - 20 Gew.-%, bevorzugt zu 3 - 15 Gew.-%, bezogen auf das Gewicht des organischen Lösemittels, aus Benzylalkohol besteht.

Umgerechnet auf die absoluten Mengen bedeutet dies, dass die erfindungsgemäßen Mittel, jeweils bezogen auf ihr Gewicht, 4,8 - 12 Gew.-%, bevorzugt 8 - 10 Gew.-% und besonders bevorzugt 8,5 - 9,6 Gew.-% Propylencarbonat sowie 0 - 2,4 Gew.-%, bevorzugt 0,18 - 2,0 Gew-%, besonders bevorzugt 1,0 - 1,8 Gew.-% Benzylalkohol enthalten. In einer besonders bevorzugten Ausführungsform enthalten die erfindungsgemäßen Die erfindungsgemäßen Mittel weder 2-Phenoxyethan-1-ol noch Benzyloxyethanol noch N-Alkylpyrrolidone.

Das erfindungsgemäße Mittel ist auf den Einsatz von direktziehenden Säurefarbstoffen zugeschnitten, dementsprechend ist der ausschließliche Einsatz von Säurefarbstoffen (d.h. Farbstoffen, die Carbonsäure- und/oder Sulfonsäuregruppierungen tragen, die auch in Form ihrer Salze eingesetzt werden können) bevorzugt. Mit anderen Worten ist es bevorzugt, wenn das erfindungsgemäße Färbemittel
- keine nichtionischen Nitrofarbstoffe
- keine nichtionischen Azofarbstoffe
- keine nichtionischen Anthrachinonfarbstoffe
- keine kationischen Anthrachinonfarbstoffe und
- keine kationischen Triarylmethanfarbstoffe enthalt.

Ein bevorzugtes erfindungsgemäßes Mittel ist weiterhin dadurch gekennzeichnet, dass es - bezogen auf sein Gewicht - weniger als 0,4 Gew.-%, bevorzugt weniger als 0,25 Gew.-%, weiter bevorzugt weniger als 0,1 Gew.-% und besonders bevorzugt weniger als 0,05 Gew.-% nichtionische Farbstoffe enthält.

Unter nichtionischen Farbstoffen werden in diesem Zusammenhang die Farbstoffe verstanden, deren Struktur keine Säuregruppe (-COOH), keine Carboxygruppe (-COO⁻), keine Suifonsäuregruppe (-SO₃H) und keine Sulfonatgruppe (-SO₃⁻) besitzt und deren Struktur darüber hinaus auch keine funktionelle Gruppe mit permanenter kationischer Ladung (wie beispielsweise eine quartare Ammoniumgruppe) besitzt.

Ein bevorzugtes erfindungsgemäßes Mittel ist weiterhin dadurch gekennzeichnet, dass es die nichtionischen Farbstoffe aus der Gruppe HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, sowie 1,4-Diamino-2-nitrobenzol, 2-Amino-4-nitrophenol, 1,4-Bis-(2-hydroxyethyl)amino-2-nitrobenzol, 3-Nitro-4-(2-hydroxyethyl)aminophenol, 2-(2-Hydroxyethyl)amino-4,6-dinitrophenol, 4-[(2-HydroXyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl) amino-4-nitrobenzol, 2-[(4-Amino-2-nitrophenyl)amino]benzoesäure, 6-Nitro-1,2,3.4-tetrahydrochinoxalin, 2-Hydroxy-1,4-naphthochinon, Pikraminsäure und deren Salze, 2-Amino-6-chtoro-4-nitropheno), 4-Ethylamino-3-nitrobenzoesäure und 2-Chlor-6-ethylamino-4-nitrophenol in einer Gesamtmenge von weniger als 0,4 Gew.-%, bevorzugt von weniger als 0,25 Gew.-%, weiter bevorzugt von weniger als 0,1 Gew.-% und besonders bevorzugt von weniger als 0,05 Gew.-%, jeweils bezogen auf das Gewicht des Mittels, enthält.

Weiterhin ist ein bevorzugtes erfindungsgemäßes Mittel dadurch gekennzeichnet, dass es, bezogen auf sein Gewicht, weniger als 0,4 Gew,-%, bevorzugt weniger als 0,25 Gew,-%, weiter bevorzugt weniger als 0,1 Gew.-% und besonders bevorzugt weniger als 0,05 Gew.-% kationische Farbstoffe enthalt.

Ein bevorzugtes erfindungsgemäßes Mittel ist weiterhin dadurch gekennzeichnet, dass es die kationischen direktziehenden Farbstoffe aus der Gruppe Basic Blue 7, Basic Blue 26, Basic Violet 2, Basic Violet 14, Basic Yellow 57, Basic Red 76, Basic Blue 99, Basic Brown 16, Basic Brown 17, HC Blue 16, Basic Yellow 87, Basic Orange 31 und Basic Red 51 in einer Gesamtmenge von weniger als 0,4 Gew.-%, bevorzugt von weniger als 0,25 Gew.-%, weiter bevorzugt von weniger als 0,1 Gew.-% und besonders bevorzugt von weniger als 0,05 Gew.-%, jeweils bezogen auf das Gewicht des Mittels, enthalt.

Bevorzugt liegt das erfindungsgemäße Mittel als tensidhaltige Lösung, wie beispielsweise als Shampoo oder Conditioner, vor, die durch Eintrag von Luft oder einem Treibmittel, wie beispielsweise LPG oder Dimethylether, aufgeschäumt werden kann,

Tenside und Emulgatoren im Sinne der vorliegenden Anmeldung sind amphiphile (bifunktionelle) Verbindungen, die aus mindestens einem hydrophoben und mindestens einem hydrophilen Molekülteil bestehen. Der hydrophobe Rest ist bevorzugt eine Kohlenwasserstoffkette mit 8-28 Kohlenstoff-Atomen, die gesättigt oder ungesättigt, linear oder verzweigt sein kann. Besonders bevorzugt ist diese C₈-C₂₈-Alkylkette linear. Basiseigenschaften der Tenside und Emulgatoren sind die orientierte Absorption an Grenzflächen sowie die Aggregation zu Mizellen und die Ausbildung von lyotropen Phasen.

Bevorzugt enthalten die erfindungsgemäßen Mittel daher zusätzlich mindestens ein kationisches Tensid in einer Gesamtmenge von 0,1 - 2 Gew.-%, bevorzugt 0,2 - 1,5 Gew.-%, besonders bevorzugt 0,4 - 0,8 Gew.-%, jeweils bezogen auf das Gewicht des Mittels.

Erfindungsgemäß bevorzugt sind kationische Tenside vom Typ der quartären Ammoniumverbindungen, der Esterquats und der Alkylamidoamine. Bevorzugte quaternäre Ammoniumverbindungen sind Ammoniumhalogenide, wie Alkyltrimethylammoniumchloride, Dialkyldimethylammoniumchloride, Trialkylmethylammoniumchloride, sowie die unter den INCI-Bezeichnungen Quaternium-27 und Quaternium-83 bekannten Imidazolium-Verbindungen. Weitere bevorzugte quaternäre Ammoniumverbindungen sind Tetraalkylammoniumsalze, wie insbesondere das unter der INCI-Bezeichnung bekannte Quaternium-52, ein Poly(Oxy-1,2-F-thanediyl), ((Octadecylnitrilio)tri-2,1-Ethanediyl)tris(Hydroxy)-Phosphat (1:1)-Salz, das die allgemeine Strukturformel (III) aufweist, worin x + y + z = 10 sind

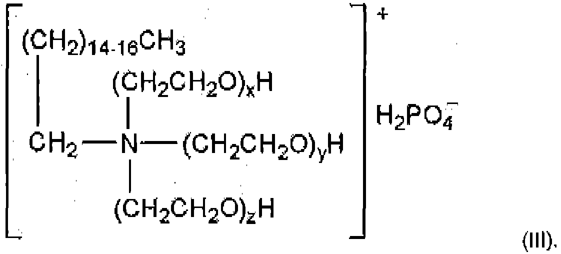

Die langen Alkylketten der oben genannten Tenside weisen bevorzugt 10 bis 22, besonders bevorzugt 12 bis 18, Kohlenstoffatome, auf. Besonders bevorzugt sind Behenyltrimethylammoniumchlorid, Stearyltrimethylammoniumchlorid und Cetyltrimethylammoniumchlorid, wobei Cetyltrimethylammoniumchlorid außerordentlich bevorzugt ist. Weitere erfindungsgemäß geeignete kationische Tenside sind quaternisierte Proteinhydrolysate, Alkylamidoamine werden üblicherweise durch Amidierung natürlicher oder synthetischer Fettsäuren und Fettsäureschnitte mit Dialkylaminoaminen hergestellt. Eine erfindungsgemäß geeignete Verbindung aus dieser Substanzgruppe stellt Tegoamid® S 18 (Stearamidopropyldimethylamin) dar. Bei Esterquats handelt es sich um Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden unter den Warenzeichen Stepantex, Dehyquart und Armocare vertrieben.

In Bezug auf optimale Schaumeigenschaften und optimale Färbeergebnisse haben sich C10-C22-Alkyltrimethylammoniumchloride und Quaternium-52 sowie Mischungen dieser Tenside als besonders gut geeignet herausgestellt. Besonders bevorzugte erfindungsgemäße Mittel sind daher dadurch gekennzeichnet, dass sie mindestens ein kationisches Tensid in einer Gesamtmenge von 0,1 - 2 Gew.-%. bevorzugt 0,2 - 1,5 Gew.-%, besonders bevorzugt 0,4 - 0,8 Gew.-%, jeweils bezogen auf das Gewicht des Mittels, enthalten, wobei mindestens ein Tensid, ausgewählt aus C10-C22-Alkyltrimethylammo-niumchloriden, insbesondere ausgewählt aus Behenyltrimethylammoniumchlorid, Stearyltrimethylammoniumchlorid und Cetyltrimethylammoniumchlorid, und Quaternium-52 sowie Mischungen dieser Tenside, enthalten sind. Außerordentlich bevorzugte erfindungsgemäße Mittel enthalten Cetyltrimethylammoniumchlorid und Quaternium-52 in einer Gesamtmenge von 0,1 - 2 Gew.-%. bevorzugt 0,2 - 1,5 Gew.-%, besonders bevorzugt 0,4 - 0,8 Gew -%, jeweils bezogen auf das Gewicht des Mittels.

Erfindungsgemäß Mittel sind dadurch gekennzeichnet, dass sie mindestens ein nichtionisches ethoxyliertes Tensid in einer Gesamtmenge von 0,1 - 1 Gew.-%, bevorzugt 0,2 - 0,8 Gew.-%, besonders bevorzugt 0,3 - 0,7 Gew.-%, jeweils bezogen auf das Gewicht des Mittels, enthalten.

Nichtionische Tenside enthalten als hydrophile Gruppe z. B. eine Polyolgruppe, eine Polyalkylenglykolethergruppe oder eine Kombination aus Polyol- und Polyglykolethergruppe. Als bevorzugte nichtionische Tenside haben sich die Ethylenoxid-Anlagerungsprodukte an gesättigte oder ungesättigte, lineare oder verzweigte Fettalkohole und Fettsäuren sowie an Glycerinfettsäureester mit jeweils 2 bis 60 Mol Ethylenoxid pro Mol Fettalkohol bzw. Fettsäure bzw. Glycerinfettsäureester sowie Mischungen hiervon erwiesen. Erfindungsgemäß besonders bevorzugt sind gesättigte oder ungesättigte C₁₀-C₂₂-Fettalkohole mit jeweils 10 bis 25 Mol Ethylenoxid pro Mol Fettalkohol (z.B. Ceteareth-20 oder Ceteareth-25). Außerordentlich bevorzugte nichtionische Tenside sind ausgewählt aus PEG-40 Hydrogenated Castor Oil und PEG-60 Hydrogenated Castor Oil sowie Mischungen hiervon. Diese besonders bevorzugten nichtionischen Tenside sind hervorragend in der Lage, die organische Lösemittelphase zu solubilisieren und tragen so positiv zum Färbeergebnis wie auch zum Schaumverhalten bei. Auf optional vorhandenes Parfüm, das bevorzugt in einer Gesamtmenge von 0,05 bis 0,5 Gew.-%, bezogen auf das Gewicht des Mittels, enthalten ist, kann so hervorragend solubilisiert werden.

Weitere erfindungsgemäß bevorzugte Mittel enthalten mindestens ein amphoteres oder zwitterionisches Tensid oder Mischungen hiervon.

Als zwitterionische Tenside werden solche oberflächenaktiven Verbindungen bezeichnet, die im Molekül mindestens eine quartäre Ammoniumgruppe und mindestens eine Carboxylat-, Sulfonat- oder SulfatGruppe tragen. Besonders geeignete zwitterionische Tenside sind die so genannten Betaine wie die N-Alkyl-N,N-dimethylammonium-glycinate, N-Acyl-aminopropyl-N.N-dimethylammoniumglycinate und 2-Alkyl-3-carboxymethyl-3-hydroxyethyl-imidazoline sowie Kokosacylaminoethyl hydroxyethylcarboxymethylglycinat. Ein bevorzugtes zwitterionisches Tensid ist das unter der INCI-Bezeichnung Cocamidopropyl Betaine bekannte Fettsäureamid-Derivat. In einer weiteren Ausführungsform der vorliegenden Erfindung enthält das Mittel weiterhin mindestens ein amphoteres Tensid. Unter amphoteren Tensiden werden solche oberflächenaktiven Verbindungen verstanden, die außer einer C₆-C₂₄-Alkyl- oder -Acylgruppe im Molekül mindestens eine freie Aminogruppe und mindestens eine COOH- oder SO₃H-Gruppe enthalten und zur Ausbildung innerer Salze befähigt sind. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside werden unter der INCI-Bezeichnung Disodium Cocoamphodipropionate mit den Handelsnamen Miranol C2M SF conc. (Rhodia), Amphoterge K-2 (Lonza) und Monateric CEM-38 (Unichema) und Bezeichnung Disodium Cocoamphodiacetate mit den Handelsnamen Dehyton (Cognis), Miranol C2M (Rhodia) und Ampholak XCO 30 (Akzo Nobel) vermarktet.

Optional können die erfindungsgemäßen Mittel auch mindestens ein anionisches Tensid enthalten. Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit etwa 8 bis 30 C-Atomen, bevorzugt 8 bis 24 C-Atomen. Zusätzlich können im Molekül Glykol- oder Polyglykolether-Gruppen, Ester-, Ether- und Amidgruppen sowie Hydroxylgruppen enthalten sein. Bevorzugte anionische Tenside sind Seifen, Alkylsulfate, Alkylethersulfate und Ethercarbonsäuren mit 8 bis 22 C-Atomen in der Alkylgruppe und bis zu 12 Glykolethergruppen im Molekül.

Da allerdings erfindungsgemäß ein Gehalt an kationischen Tensiden, wie vorstehend beschrieben, bevorzugt ist und es bei gleichzeitiger Anwesenheit von kationischen und anionischen Tensiden zu unerwünschten Wechselwirkung, beispielsweise Agglomeratbildung, kommen kann, sind besonders bevorzugte erfindungsgemäße Mittel dadurch gekennzeichnet, dass keine anionischen Tenside enthalten sind.

Weitere erfindungsgemäß besonders bevorzugte Mittel sind durch einen Gesamttensidgehalt von 0,5 - 2 Gew.-%, bevorzugt 0,7 - 1,5 Gew.-%, besonders bevorzugt 0,8 - 1,2 Gew.-%, jeweils bezogen auf das Gewicht des Mittels, gekennzeichnet.

Weitere erfindungsgemäß besonders bevorzugte Mittel können ein oder mehrere kationische Polymere aus der Gruppe Polyquaternium-1, Polyquaternium-2, Polyquaternium-3, Polyquaternium-4, Polyquaternium-5, Polyquaternium-6, Polyquaternium-7, Polyquaternium-8, Polyquaternium-9, Polyquaternium-10, Polyquaternium-11, Polyquaternium-14, Polyquaternium-16, Polyquaternium-17, Polyquaternium-18, Polyquaternium-22, Polyquaternium-24, Polyquaternium-27, Polyquaternium-28, Polyquaternium-32, Polyquaternium-33, Polyquaternium-37, Polyquaternium-39, Polyquaternium-44, Polyquaternium-46, Polyquaternium-53, Polyquaternium-55, Polyquarternium-64, Polyquaternium-67, Polyquaternium-68, Polyquaternium-69 und/oder Polyquaternium-86, sowie Mischungen hiervon, enthalten, bevorzugt in einer Gesamtmenge von 0,01 bis 2 Gew.-%, bevorzugt 0,1 - 1,5 Gew.-%, besonders bevorzugt 0,5 - 1,0 Gew.-%, jeweils bezogen auf das Gewicht des Mittels, Besonders bevorzugte erfindungsgemäße Mittel sind dadurch gekennzeichnet, dass sie eine Mischung von Polyquaternium-4 und Polyquaternium-11 enthalten, außerordentlich bevorzugt in einer Gesamtmenge von 0,01 bis 2 Gew.-%, bevorzugt 0,1 -1,5 Gew.-%, besonders bevorzugt 0,5 - 1,0 Gew.-%, jeweils bezogen auf das Gewicht des Mittels. Die Gegenwart von mindestens einem der genannten kationischen Polymere kann sich förderlich auf den Farbaufzug und/oder die gewünschten Schaumeigenschaften auswirken.

Weiterhin wurde überraschend festgestellt, dass sich die Gegenwart von mindestens einem nichtionischen Polyvinylpyrrolidon-/Vinylacetat-Polymer in einer Gesamtmenge von 0,1 -2,0 Gew.-%, bevorzugt 0,2 - 1,5 Gew,-%, besonders bevorzugt 0,4 - 1,0 Gew.-%, jeweils bezogen auf das Gewicht des Mittels, förderlich auf den Farbaufzug und/oder die gewünschten Schaumeigenschaften auswirken kann. Weitere bevorzugte erfindungsgemäße Mittel sind daher dadurch gekennzeichnet, dass sie mindestens ein nichtionisches Polyvinylpyrrolidon-/Vinylacetat-Polymer in einer Gesamtmenge von 0,1 - 2,0 Gew.-%, bevorzugt 0,2 - 1,5 Gew.-%, besonders bevorzugt 0,4 - 1,0 Gew.-%, jeweils bezogen auf das Gewicht des Mittels, enthalten.

Da die erfindungsgemäßen Mittel sprühbar sein sollen, enthalten sie in einer außerordentlich bevorzugten Ausführungsform keine Fettkomponenten mit einem Schmelzpunkt im Bereich von 28 - 80°C. Beispiele für erfindungsgemäß unerwünschte Fettkomponenten mit einem Schmelzpunkt im Bereich von 28 - 80°C sind lineare gesättigte 1-Alkanole mit 12 - 30 Kohlenstoffatomen und Ester aus ein- und mehrwertigen C1-C10-Aikanolen und C8-C30-Alkansäuren, weiterhin Wachse. Die vorstehend genannten nichtionischen Tenside können Schmelzpunkte im Bereich von 28 - 80°C aufweisen, zählen jedoch nicht zu den vorstehend genannten Fettkomponenten. Die vorstehend genannten Fettkomponenten mit einem Schmelzpunkt im Bereich von 28 - 80°C unterscheiden sich von den nichtionischen Tensiden durch die Abwesenheit hydrophiler Gruppen.

Die erfindungsgemäßen Mittel weisen bevorzugt eine Viskosität im Bereich von 50 - 1000 mPas, besonders bevorzugt 100 - 700 mPas, außerordentlich bevorzugt 150 - 300 mPas, auf, jeweils gemessen bei 20°C im Haake-Viskosimeter Typ MV2 mit einer Geschwindigkeit von 8 Umdrehungen/Minute.

Negative Auswirkungen auf das Schaumverhalten wurden ebenfalls für aliphatische C1-C6-Alkohole, wie Ethanol, Isopropanol, für Polyole, ausgewählt aus C₂ - C₉-Alkanolen mit 2 - 6 Hydroxylgruppen, beispielsweise Ethylenglycol, Glycerin, Propylenglycol, 1,3-Butylenglycol, und Polyethylenglycolen mit mindestens 2 Ethylenoxid-Einheiten, beispielsweise PEG-3, PEG-4, PEG-6, PEG-7, PEG-8, usw. beobachtet. Erfindungsgemäße Mittel enthalten daher keine aliphatischen C1-C6-Alkohole, keine C₂ - C₉-Alkanole mit 2 - 6 Hydroxylgruppen und keine Polyethylenglycole mit mindestens 2 Ethylenoxid-Einheiten. Die hier ausgeschlossenen Polyethylenglycole mit mindestens 2 Ethylenoxid-Einheiten bestehen nur aus Ethylenglycol-Einheiten und enthalten keine hydrophoben Molekülteile.

Auch wenn die Mittel erfindungsgemäß auf einen sauren pH-Wert eingestellt werden, kann zur Feineinstellung des pH-Wertes trotzdem der Einsatz geringer Mengen Alkalisierungsmittel notwendig werden. Die zur Einstellung der bevorzugten pH-Werte gegebenenfalls verwendbaren Alkalisierungsmittel können aus der Gruppe, die gebildet wird aus Ammoniak, Alkanolaminen, basischen Aminosäuren, sowie anorganischen Alkalisierungsmitteln wie (Erd-)Alkalimetallhydroxiden, (Erd-)Alkalimetallmetasilikaten, (Erd-)Alkalimetallphosphaten und (Erd-)Alkalimetallhydrogenphosphaten, ausgewählt werden. Bevorzugte anorganische Alkalisierungsmittel sind Natriumhydroxid, Kaliumhydroxid, Natriumsilicat und Natriummetasilicat. Erfindungsgemäß einsetzbare, organische Alkalisierungsmittel werden bevorzugt ausgewählt aus Monoethanolamin, 2-Amino-2-methylpropanol und Triethanolamin. Die als erfindungsgemäßes Alkalisierungsmittel einsetzbaren basischen Aminosäuren werden bevorzugt ausgewählt aus der Gruppe, die gebildet wird aus Arginin, Lysin, Ornithin und Histidin, besonders bevorzugt Arginin. Es hat sich aber im Rahmen der Untersuchungen zur vorliegenden Erfindung herausgestellt, dass weiterhin erfindungsgemäß bevorzugte Mittel dadurch gekennzeichnet sind, dass sie zusätzlich ein organisches Alkalisierungsmittel enthalten. Eine Ausführungsform des ersten Erfindungsgegenstands ist dadurch gekennzeichnet, dass das Mittel zusatzlich mindestens ein Alkalisierungsmittel enthält, das ausgewählt ist aus der Gruppe, die gebildet wird aus Ammoniak, Alkanolaminen und basischen Aminosäuren, insbesondere aus Ammoniak, Monoethanolamin und Arginin oder seinen kosmetisch verträglichen Salzen. Ein zweiter Gegenstand der vorliegenden Erfindung ist ein Haarfärbeprodukt, umfassend eine Spraydose und, darin verpackt, ein erfindungsgemäßes oder erfindungsgemäß bevorzugtes Färbemittel nach einem der Ansprüche 1 bis 10 sowie mindestens ein Treibmittel, wobei das Gewichtsverhältnis von Färbemittel zu Treibmittel im Bereich von 5:95 bis 95:5, bevorzugt 50:50, besonders bevorzugt 80:20, liegt.

Bevorzugte Treibmittel (Treibgase) sind Propan, Propen, n-Butan, iso-Butan, iso-Buten, n-Pentan, Penten, iso-Pentan, iso-Penten, Methan, Ethan, Dimethylether, Stickstoff, Luft, Sauerstoff, Lachgas, 1,1,1,3-Tetrafluorethan, Heptafluoro-n-propan, Perfluorethan, Monochlordifluormethan, 1,1-Difluorethan, und zwar sowohl einzeln als auch in Kombination. Auch hydrophile Treibgase, wie z. B. Kohlendioxid, können vorteilhaft im Sinne der vorliegenden Erfindung eingesetzt werden, wenn der Anteil an hydrophilen Gasen gering gewählt wird und lipophiles Treibgas (z. B. Propan/Butan) im Überschuss vorliegt. Besonders bevorzugt sind Propan, n-Butan, iso-Butan sowie Mischungen dieser Treibgase. Besonders bevorzugte Treibgasmischungen sind LPG ("Liquefied Petroleum Gas").

Als Beispiel für erfindungsgemäße Spender mit integrierter Druckquelle sind Druckbehälter zu nennen, die üblicherweise im Behälterinneren entweder einen geeigneten Druckspeicher aufweisen, z. B. einen mechanischen, oder aber ein Treibmittel enthalten und auf diesem Wege das Innere des Behälters unter Druck setzen. Derartige Druckbehälter verfügen üblicherweise über geeignete Ventilvorrichtungen zur Ausgabe der im Inneren des Druckbehälters befindlichen Zubereitung während einer entsprechenden Ventilbetätigung. Solche Druckbehälter sind vor allem in Verbindung mit gasförmigen und/oder flüssigen Treibmitteln in Form von Aerosolspendern für unterschiedlichste kosmetische Anwendungen vorbekannt, z. B. Haarstylingspray, Haarfärbezubereitungen, Deospray, Rasierschaum/-gel etc..

Alternativ können erfindungsgemäß auch manuell betätigte Spender zum Einsatz kommen, die sich lediglich der Krafteinwirkung des Nutzers bedienen, um eine schaumförmige Zubereitungsabgabe zu bewirken. Bei diesen Bauformen kann vorteilhaft auf eine zusätzliche Druckquelle, z. B. Treibmittel, verzichtet werden, was vor allem aus Kosten- sowie Nachhaltigkeitsgründen wünschenswert ist. Solche durch manuelle Krafteinwirkung betätigbaren Schaumspender sorgen neben der Förderung der Haarfärbezubereitung aus dem Vorratsbehälter zur Abgabeöffnung hin parallel auch für eine entsprechende Aufschäumung der Haarfärbezubereitung. Während dieser Aufschäumung oder Schaumbildung wird grundsätzlich die Haarfärbezubereitung mit einer gasförmigen Komponente, insbesondere Luft, vermischt. Im Einzelnen ist dazu eine Schäumvorrichtung vorgesehen, die dies bewerkstelligt.

Gemäß einer ersten Variante eines manuell betätigbaren Spenders ist dieser als Schüttelspender ausgeführt, mit mindestens einem Vorratsbehälter zur Aufnahme der Haarfärbezubereitung und einer zugehörigen Abgabevorrichtung zur schaumförmigen Abgabe der Haarfärbezubereitung. Dabei ist die Abgabevorrichtung, insbesondere lösbar, mit dem Vorratsbehälter verbunden. Die eigentliche Schaumbildung erfolgt innerhalb des Schüttelspenders durch Agitation der Haarfärbezubereitung innerhalb des Vorratsbehälters. Insofern bildet der Schüttelspender in Verbindung der entsprechenden Spenderbewegung die oben genannte Schäumvorrichtung. Im Anschluss an diese Art der Aufschäumung kann dann die Abgabe der schaumförmigen Haarfärbezubereitung mittels der Abgabevorrichtung erfolgen. Eine weitere sinnvolle Spendervariante ergibt sich durch Ausgestaltung als Quetsch- oder Squeeze-Schaumspender. Ein solcher Quetsch-Schaumspender besitzt neben dem mindestens einen Vorratsbehälter zur Aufnahme der Haarfärbezubereitung eine entsprechende Applikationsvorrichtung, innerhalb der die Aufschäumung sowie anschließende Abgabe der Haarfärbezubereitung erfolgt. Die eigentliche Förderung der Haarfärbezubereitung aus dem Vorratsbehälter wird mittels Krafteinwirkung auf die flexible Vorratsbehälterwandung bewirkt. Dabei sorgt die reversible Verformung der Vorratsbehälterwandung für einen Druckaufbau im Inneren des Vorratsbehälters, so dass als Folge die Haarfärbezubereitung aus dem Vorratsbehälter getrieben wird. Dazu ist es notwendig, die Vorratsbehälterwandung hinreichend flexibel bzw. reversibel verformbar zu gestalten. Dies wird gewahrleistet durch eine anwendungsbezogen zielgerichte Auslegung der Dicke der Vorratsbehälterwandung in Verbindung mit einer geeigneten Materialauswahl für die Vorratsbehälterwandung. Bevorzugt wird die Vorratsbehälterwandung eines entsprechenden Quetsch-Schaumspenders aus einem Polyolefin, wie beispielsweise Polypropylen (PP), high density Polyethylen (HDPE), medium density Polyethylen (MDPE), low density Polyethylen (LDPE), linear low density Polyethylen (LLDPE), ausgeführt. Darunter ist Polypropylen (PP) bevorzugt geeignet.

Die Applikationsvorrichtung eines solchen Quetsch-Schaumspenders umfasst zur Aufschäumung der Haarfärbezubereitung auch eine entsprechende Schäumvorrichtung. Die Schäumvorrichtung ist in der Lage, eine Zubereitungsmenge mit einer Gasmenge im geeigneten Dosierverhältnis zu vermischen, um die gewünschte Schaumkonsistenz der Haarfärbezubereitung auszubilden. Üblicherweise wird hierzu ein eingezogener Zubereitungsstrom mit einem eingezogenen Gasstrom innerhalb einer Mischkammer der Schäumvorrichtung zusammen geführt und dort durch strömungstechnische Verwirbelung vermengt. Besonders bevorzugt wird als gasförmige Komponente zur Schaumbildung Luft verwendet, die entweder unmittelbar aus der Vorratsbehälter oder der Umgebung eingezogen wird.

Die grundsätzliche Funktionsweise derartiger Quetsch-Schaumspender wird auch in den Dokumenten WO 2007/086730 A2/A3 sowie EP 1237660 B1 beschrieben. Ein erfindungsgemäßer Quetsch-Schaumspender kann auch entsprechend zu diesen Patentdokumenten ausgebildet sein. Insbesondere kann der erfindungsgemäße Quetsch-Schaumspender gemäß der Offenbarung von EP 1237660 B1 so ausgeführt werden, dass eine Verwendung des Quetsch-Schaumspenders in im Wesentlichen aufrechter Position als auch in Überkopfstellung möglich ist.

Analog kann der Spender auch als Pump-Schaumspender ausgebildet sein mit mindestens einer Vorratsbehälter zur Aufnahme der Haarfärbezubereitung sowie einer Applikationsvorrichtung, wobei in diesem Fall die Applikationsvorrichtung eine Pumpvorrichtung zur Förderung sowohl der Haarfärbezubereitung als der gasförmigen Komponente, bevorzugt Luft, aufweist und darüber hinaus eine entsprechende Schäumvorrichtung umfasst. Im Detail ergibt sich die Funktionsweise und der konstruktive Aufbau derartiger Pump-Schaumspender u, a. auch aus den Patentdokumenten WO 2007/083206 A1 oder WO 2007/091882 A1. Insbesondere kann der erfindungsgemäße Pump-Schaumspender gemäß der Offenbarung dieser genannten Dokumente ausgebildet sein.

Bezüglich weiterer bevorzugter Ausführungsformen des erfindungsgemäßen Haarfärbeprodukts gilt mutatis mutandis das zu den erfindungsgemäßen und erfindungsgemäß bevorzugten Mitteln Gesagte. Die erfindungsgemäßen oder erfindungsgemäß bevorzugten Färbemittel können allerdings auch vorteilhaft aus einem Nichtaerosolschaumspender als Schaum ausgetragen und in einem erfindungsgemäßen Haarfärbeverfahren verwendet werden. Ein weiterer Gegenstand der vorliegenden Erfindung ist daher ein Haarfärbeprodukt, umfassend einen Nichtaerosolschaumspender und, darin verpackt, ein erfindungsgemäßes oder erfindungsgemäß bevorzugtes Färbemittel nach einem der Ansprüche 1 - 10. Ein dritter Gegenstand der vorliegenden Erfindung ist ein Verfahren zum Färben von keratinischen Fasern, bei dem ein Mittel gemäß einem der Ansprüche 1 bis 10 aus einem Spender, bevorzugt aus einer Spraydose gemäß Anspruch 11 oder aus einem Nichtaerosolschaumspender, als Schaum ausgebracht wird, anschließend der so erhaltene Schaum auf den keratinischen Fasern verteilt wird, dann für eine Zeit von 30 Sekunden bis 60 Minuten, vorzugsweise von 5 bis 45 Minuten, auf den keratinischen Fasern verbleibt und anschließend aus den keratinischen Fasern ausgewaschen wird.

Bezüglich weiterer bevorzugter Ausführungsformen des erfindungsgemäßen Verfahrens gilt mutatis mutandis das zu den erfindungsgemäßen und erfindungsgemäß bevorzugten Mitteln Gesagte.

### 1. Formulierungsbeispiele

Es wurden die folgenden Formulierungen hergestellt - alle Angaben erfolgen, sofern nicht anders angegeben, in Gewichtsprozent Aktivsubstanz. Die mit "V" bezeichneten Proben sind Vergleichszusammensetzungen; die mit "E" bezeichneten Proben sind erfindungsgemäß.

**Färbemittel**

| Inhaltsstoff | V1 (Gew.-%) | E1 (Gew.-%) | E2 (Gew.-%) | E3 (Gew.-%) | V2 (Gew.-%) | V3 (Gew.-%) | V4 (Gew.-%) |
|---|---|---|---|---|---|---|---|
| Acid Black No. 1 | 0,2 | 0,2 | 0,2 | 0,2 | 0,2 | 0,2 | 0,2 |
| Acid Orange 7 | 0,2 | 0,2 | 0,2 | 0,2 | 0,2 | 0,2 | 0,2 |
| Acid Violet 43 | 0,1 | 0,1 | 0,1 | 0,1 | 0,1 | 0,1 | 0,1 |
| Benzylalkohol | 10 | - | 1 | 2 | 3 | 4 | 5 |
| Propylencarbonat | - | 10 | 9 | 8 | 7 | 6 | 5 |
| Polyquaternium-4 | 0,319 | 0,319 | 0,319 | 0,319 | 0,319 | 0,319 | 0,319 |
| PVP/VA-Copolymer (60/40) | 0,75 | 0,75 | 0,75 | 0,75 | 0,75 | 0,75 | 0,75 |
| Polyquaternium-11 | 0,3 | 0,3 | 0,3 | 0,3 | 0,3 | 0,3 | 0,3 |
| Natriumbenzoat | 0,319 | 0,319 | 0,319 | 0,319 | 0,319 | 0,319 | 0,319 |
| Quaternium-52 | 0,213 | 0,213 | 0,213 | 0,213 | 0,213 | 0,213 | 0,213 |
| Cetyltrimonium chlorid | 0,27 | 0,27 | 0,27 | 0,27 | 0,27 | 0,27 | 0,27 |
| Milchsäure | 0,08 | 0,08 | 0,08 | 0,08 | 0,08 | 0,08 | 0,08 |
| PEG 40-Hydrogenated Castor Oil | 0,425 | 0,425 | 0,425 | 0,425 | 0,425 | 0,425 | 0,425 |
| Parfüm | 0,2 | 0,2 | 0,2 | 0,2 | 0,2 | 0,2 | 0,2 |
| Wasser (dest.) | ad 100 | ad 100 | ad 100 | ad 100 | ad 100 | ad 100 | ad 100 |
| Schaumstabilität | schlecht | gut | gut | gut | schlecht | schlecht | schlecht |

### 2. Anwendung

Jedes Färbemittel wurde aus einem Nonaerosolspender bzw. aus einem Treibgasspender als Schaum ausgegeben und auf vorgeschädigte Haarsträhnen (Kerling 6-0, vorgeschädigt) appliziert (4 g Färbemittel pro 1 g Haar), dort für 40 Minuten belassen und anschließend mit Wasser (32 °C) ausgewaschen. Dann wurden die Strähnen für 24 Stunden bei 25 °C und 25 %rel. Luftfeuchte gelagert.

Alle Strähnen zeigten hervorragende Färbeeigenschaften.

## Patentansprüche

1. Wasserhaltiges kosmetisches Mittel zum Färben von keratinischen Fasern, welches
- einen pH-Wert im Bereich von 1,0 bis 5,5 besitzt und
- mindestens einen direktziehenden Säurefarbstoff und
- bezogen auf das Gewicht des Mittels, 6 bis 12 Gew.-% organisches Lösemittel, das zu 80 - 100 Gew.-% aus Propylencarbonat und zu 0 - 20 Gew.-% aus Benzylalkohol besteht,
- und mindestens ein nichtionisches ethoxyliertes Tensid in einer Gesamtmenge von 0,1 - 1 Gew.-%, bezogen auf das Gewicht des Mittels,
enthält,
wobei der Gesamtgehalt an organischem Lösemittel 6 bis 12 Gew.-%, bezogen auf das Gewicht des Mittels, beträgt.

2. Mittel nach Anspruch 1, **dadurch gekennzeichnet, dass** es mindestens einen direktziehenden Säurefarbstoff aus der Gruppe Acid Yellow 1, Acid Yellow 3, Acid Yellow 9, Acid Yellow 17, Acid Yellow 23, Acid Yellow 36, Acid Yellow 121, Acid Orange 6, Acid Orange 7, Acid Orange 10, Acid Orange 11, Acid Orange 15, Acid Orange 20, Acid Orange 24, Acid Red 14, Acid Red 18, Acid Red 27, Acid Red 33, Acid Red 35, Acid Red 51, Acid Red 52, Acid Red 73, Acid Red 87, Acid Red 95, Acid Red 184, Acid Red 195, Acid Violet 43, Acid Violet 49, Acid Violet 50, Acid Blue 1, Acid Blue 3, Acid Blue 7, Acid Blue 104, Acid Blue 9, Acid Blue 62, Acid Blue 74, Acid Blue 80, Acid Green 3, Acid Green 5, Acid Green 9, Acid Green 22, Acid Green 25, Acid Green 50, Acid Black 1, Acid Black 52, Food Yellow 8, Food Blue 5, D&C Yellow 7, D&C Yellow 8, D&C Orange 4, D&C Green 5, D&C Orange 10, D&C Orange 11, D&C Red 21, D&C Red 27, D&C Red 33, D&C Violet 2 und/oder D&C Brown 1 enthält.

3. Mittel nach einem der Ansprüche 1 oder 2, **dadurch gekennzeichnet, dass** es mindestens eine der folgenden Kombinationen aus direktziehenden Säurefarbstoffen enthält: Acid Yellow 1/ Acid Orange 7; Acid Yellow 3/ Acid Orange 7; Acid Yellow 9/ Acid Orange 7; Acid Yellow 17/ Acid Orange 7; Acid Yellow 23/ Acid Orange 7; Acid Yellow 36/ Acid Orange 7; Acid Yellow 121/ Acid Orange 7; Acid Red 14/ Acid Orange 7; Acid Red 18/ Acid Orange 7; Acid Red 27/ Acid Orange 7; Acid Red 33/ Acid Orange 7; Acid Red 35/ Acid Orange 7; Acid Red 51/ Acid Orange 7; Acid Red 52/ Acid Orange 7; Acid Red 73/ Acid Orange 7; Acid Red 87/ Acid Orange 7; Acid Red 95/ Acid Orange 7; Acid Red 184/ Acid Orange 7; Acid Red 195/ Acid Orange 7; Acid Violet 43/ Acid Orange 7; Acid Violet 49/ Acid Orange 7 oder Acid Violet 50/ Acid Orange 7.

4. Mittel nach einem der Ansprüche 1 bis 3, **dadurch gekennzeichnet, dass** es einen oder mehrere direktziehende Säurefarbstoffe in einer Gesamtmenge von 0,01 bis 5,5 Gew.-%, bevorzugt von 0,08 bis 4,7 Gew.-%, weiter bevorzugt von 0,2 bis 3,4 Gew.-% und besonders bevorzugt von 0,3 bis 1,8 Gew.-% - bezogen auf das Gewicht des Mittels - enthält.

5. Mittel nach einem der Ansprüche 1 bis 4, **dadurch gekennzeichnet, dass** es einen pH-Wert von 1,5 bis 4,7, bevorzugt von 1,6 bis 3,9, weiter bevorzugt von 1,7 bis 3,1 und besonders bevorzugt von 1,8 bis 2,5 besitzt.

6. Mittel nach einem der Ansprüche 1 bis 5, **dadurch gekennzeichnet, dass** es, jeweils bezogen auf sein Gewicht, 4,8 - 12 Gew.-%, bevorzugt 8 - 10 Gew.-% und besonders bevorzugt 8,5 - 9,6 Gew.-% Propylencarbonat sowie 0 - 2,4 Gew.-%, bevorzugt 0,18 - 2,0 Gew.-%, besonders bevorzugt 1,0 - 1,8 Gew.-% Benzylalkohol enthält.

7. Mittel nach einem der Ansprüche 1 bis 6, **dadurch gekennzeichnet, dass** es mindestens ein kationisches Tensid in einer Gesamtmenge von 0,1 - 2 Gew.-%, bevorzugt 0,2 - 1,5 Gew.-%, besonders bevorzugt 0,4 - 0,8 Gew.-%, jeweils bezogen auf das Gewicht des Mittels, enthält.

8. Mittel nach Anspruch 7, **dadurch gekennzeichnet, dass** das mindestens eine kationische Tensid ausgewählt ist aus C10-C22-Alkyltrimethylammoniumchloriden, insbesondere ausgewählt aus Behenyltrimethylammoniumchlorid, Stearyltrimethylammoniumchlorid und Cetyltrimethylammoniumchlorid, und Quaternium-52 sowie Mischungen dieser Tenside.

9. Mittel nach einem der Ansprüche 1 bis 8, **dadurch gekennzeichnet, dass** es mindestens ein nichtionisches ethoxyliertes Tensid in einer Gesamtmenge von 0,2 - 0,8 Gew.-%, besonders bevorzugt 0,3 - 0,7 Gew.-%, jeweils bezogen auf das Gewicht des Mittels, enthält,.

10. Mittel nach einem der Ansprüche 1 bis 9, **dadurch gekennzeichnet, dass** das mindestens eine nichtionische ethoxylierte Tensid ausgewählt ist aus Ethylenoxid-Anlagerungsprodukten an gesättigte oder ungesättigte, lineare oder verzweigte Fettalkohole und Fettsäuren sowie an Glycerinfettsäureester mit jeweils 2 bis 60 Mol Ethylenoxid pro Mol Fettalkohol bzw. Fettsäure bzw. Glycerinfettsäureester sowie Mischungen hiervon, insbesondere ausgewählt aus PEG-40 Hydrogenated Castor Oil und PEG-60 Hydrogenated Castor Oil sowie Mischungen hiervon.

11. Mittel nach einem der Ansprüche 1 bis 10, **dadurch gekennzeichnet, dass** es mindestens ein kationisches Polymer in einer Gesamtmenge von 0,01 bis 2 Gew.-%, bevorzugt 0,1 - 1,5 Gew.-%, besonders bevorzugt 0,5 - 1,0 Gew.-%, jeweils bezogen auf das Gewicht des Mittels, enthält.

12. Mittel nach Anspruch 11, **dadurch gekennzeichnet, dass** das kationische Polymer ausgewählt ist aus der Gruppe, bestehend aus Polyquaternium-1, Polyquaternium-2, Polyquaternium-3, Polyquaternium-4, Polyquaternium-5, Polyquaternium-6, Polyquaternium-7, Polyquaternium-8, Polyquaternium-9, Polyquaternium-10, Polyquaternium-11, Polyquaternium-14, Polyquaternium-16, Polyquaternium-17, Polyquaternium-18, Polyquaternium-22, Polyquaternium-24, Polyquaternium-27, Polyquaternium-28, Polyquaternium-32, Polyquaternium-33, Polyquaternium-37, Polyquaternium-39, Polyquaternium-44, Polyquaternium-46, Polyquaternium-53, Polyquaternium-55, Polyquarternium-64, Polyquaternium-67, Polyquaternium-68, Polyquaternium-69 und Polyquaternium-86, sowie Mischungen hiervon, besonders bevorzugt ausgewählt aus Mischungen von Polyquaternium-4 und Polyquaternium-11.

13. Mittel nach einem der Ansprüche 1 bis 12, **dadurch gekennzeichnet, dass** es mindestens ein nichtionisches Polyvinylpyrrolidon-/Vinylacetat-Polymer in einer Gesamtmenge von 0,1 - 2,0 Gew.-%, bevorzugt 0,2 - 1,5 Gew.-%, besonders bevorzugt 0,4 - 1,0 Gew.-%, jeweils bezogen auf das Gewicht des Mittels, enthält.

14. Haarfärbeprodukt, **dadurch gekennzeichnet, dass** es eine Spraydose und, darin verpackt, ein Färbemittel nach einem der Ansprüche 1 bis 13 sowie mindestens ein Treibmittel umfasst, wobei das Gewichtsverhältnis von Färbemittel zu Treibmittel im Bereich von 5:95 bis 95:5, bevorzugt 50:50, besonders bevorzugt 80:20, liegt.

15. Verfahren zum Färben von keratinischen Fasern, **dadurch gekennzeichnet, dass** ein Mittel gemäß einem der Ansprüche 1 bis 13 aus einem Spender, bevorzugt aus einer Spraydose gemäß Anspruch 11 oder aus einem Nichtaerosolschaumspender, als Schaum ausgebracht wird, anschließend der so erhaltene Schaum auf den keratinischen Fasern verteilt wird, dann für eine Zeit von 30 Sekunden bis 60 Minuten, vorzugsweise von 5 bis 45 Minuten, auf den keratinischen Fasern verbleibt und anschließend aus den keratinischen Fasern ausgewaschen wird.

## Claims

1. A water-containing cosmetic agent for dyeing keratin fibers, which
- has a pH in the range of from 1.0 to 5.5 and
- contains at least one direct acid dye and
- contains, based on the weight of the agent, 6 to 12 wt.% organic solvent consisting of 80-100 wt.% propylene carbonate and 0-20 wt.% benzyl alcohol,
- and contains at least one non-ionic ethoxylated surfactant in a total amount of 0.1-1 wt.%, based on the weight of the agent,
wherein the total content of organic solvent is 6 to 12 wt.%, based on the weight of the agent.

2. The agent according to claim 1, **characterized in that** it contains at least one direct acid dye from the group comprising Acid Yellow 1, Acid Yellow 3, Acid Yellow 9, Acid Yellow 17, Acid Yellow 23, Acid Yellow 36, Acid Yellow 121, Acid Orange 6, Acid Orange 7, Acid Orange 10, Acid Orange 11, Acid Orange 15, Acid Orange 20, Acid Orange 24, Acid Red 14, Acid Red 18, Acid Red 27, Acid Red 33, Acid Red 35, Acid Red 51, Acid Red 52, Acid Red 73, Acid Red 87, Acid Red 95, Acid Red 184, Acid Red 195, Acid Violet 43, Acid Violet 49, Acid Violet 50, Acid Blue 1, Acid Blue 3, Acid Blue 7, Acid Blue 104, Acid Blue 9, Acid Blue 62, Acid Blue 74, Acid Blue 80, Acid Green 3, Acid Green 5, Acid Green 9, Acid Green 22, Acid Green 25, Acid Green 50, Acid Black 1, Acid Black 52, Food Yellow 8, Food Blue 5, D&C Yellow 7, D&C Yellow 8, D&C Orange 4, D&C Green 5, D&C Orange 10, D&C Orange 11, D&C Red 21, D&C Red 27, D&C Red 33, D&C Violet 2 and/or D&C Brown 1.

3. The agent according to one of claims 1 or 2, **characterized in that** it contains at least one of the following combinations of direct acid dyes: Acid Yellow 1/ Acid Orange 7; Acid Yellow 3/ Acid Orange 7; Acid Yellow 9/ Acid Orange 7; Acid Yellow 17/ Acid Orange 7; Acid Yellow 23/ Acid Orange 7; Acid Yellow 36/ Acid Orange 7; Acid Yellow 121/ Acid Orange 7; Acid Red 14/ Acid Orange 7; Acid Red 18/ Acid Orange 7; Acid Red 27/ Acid Orange 7; Acid Red 33/ Acid Orange 7; Acid Red 35/ Acid Orange 7; Acid Red 51/ Acid Orange 7; Acid Red 52/ Acid Orange 7; Acid Red 73/ Acid Orange 7; Acid Red 87/ Acid Orange 7; Acid Red 95/ Acid Orange 7; Acid Red 184/ Acid Orange 7; Acid Red 195/ Acid Orange 7; Acid Violet 43/ Acid Orange 7; Acid Violet 49/ Acid Orange 7 or Acid Violet 50/ Acid Orange 7.

4. The agent according to one of claims 1 to 3, **characterized in that** it contains one or more direct acid dyes in a total amount of from 0.01 to 5.5 wt.%, preferably from 0.08 to 4.7 wt.%, more preferably from 0.2 to 3.4 wt.% and particularly preferably from 0.3 to 1.8 wt.%, based on the total weight of the agent.

5. The agent according to one of claims 1 to 4, **characterized in that** it has a pH of from 1.5 to 4.7, preferably from 1.6 to 3.9, more preferably from 1.7 to 3.1 and particularly preferably from 1.8 to 2.5.

6. The agent according to one of claims 1 to 5, **characterized in that** it contains, in each case based on its weight, 4.8-12 wt.%, preferably 8-10 wt.% and particularly preferably 8.5-9.6 wt.%, propylene carbonate and 0-2.4 wt.%, preferably 0.18-2.0 wt.%, particularly preferably 1.0-1.8 wt.%, benzyl alcohol.

7. The agent according to one of claims 1 to 6, **characterized in that** it contains at least one cationic surfactant in a total amount of 0.1-2 wt.%, preferably 0.2-1.5 wt.%, particularly preferably 0.4-0.8 wt.%, in each case based on the weight of the agent.

8. The agent according to claim 7, **characterized in that** the at least one cationic surfactant is selected from C10-C22 alkyl trimethyl ammonium chlorides, in particular selected from behenyl trimethyl ammonium chloride, stearyl trimethyl ammonium chloride and cetyl trimethyl ammonium chloride, and quaternium-52, and mixtures of these surfactants.

9. The agent according to one of claims 1 to 8, **characterized in that** it contains at least one non-ionic ethoxylated surfactant in a total amount of 0.2-0.8 wt.%, particularly preferably 0.3-0.7 wt.%, in each case based on the weight of the agent.

10. The agent according to one of claims 1 to 9, **characterized in that** the at least one non-ionic ethoxylated surfactant is selected from ethylene oxide addition products of saturated or unsaturated, linear or branched fatty alcohols and fatty acids and of glycerol fatty acid esters with in each case 2 to 60 mol ethylene oxide per mol fatty alcohol or fatty acid or glycerol fatty acid ester and mixtures thereof, in particular selected from PEG-40 Hydrogenated Castor Oil and PEG-60 Hydrogenated Castor Oil and mixtures thereof.

11. The agent according to one of claims 1 to 10, **characterized in that** it contains at least one cationic polymer in a total amount of from 0.01 to 2 wt.%, preferably 0.1-1.5 wt.%, particularly preferably 0.5-1.0 wt.%, in each case based on the weight of the agent.

12. The agent according to claim 11, **characterized in that** the cationic polymer is selected from the group consisting of polyquaternium-1, polyquaternium-2, polyquaternium-3, polyquaternium-4, polyquaternium-5, polyquaternium-6, polyquaternium-7, polyquaternium-8, polyquaternium-9, polyquaternium-10, polyquaternium-11, polyquaternium-14, polyquaternium-16, polyquaternium-17, polyquaternium-18, polyquaternium-22, polyquaternium-24, polyquaternium-27, polyquaternium-28, polyquaternium-32, polyquaternium-33, polyquaternium-37, polyquaternium-39, polyquaternium-44, polyquaternium-46, polyquaternium-53, polyquaternium-55, polyquaternium-64, polyquaternium-67, polyquaternium-68, polyquaternium-69 and polyquaternium-86, and mixtures thereof, particularly preferably selected from mixtures of polyquaternium-4 and polyquaternium-11.

13. The agent according to one of claims 1 to 12, **characterized in that** it contains at least one non-ionic polyvinylpyrrolidone-vinyl acetate polymer in a total amount of 0.1-2.0 wt.%, preferably 0.2-1.5 wt.%, particularly preferably 0.4-1.0 wt.%, in each case based on the weight of the agent.

14. A hair dye product, **characterized in that** it comprises a spray can and, packaged therein, a coloring agent according to one of claims 1 to 13 and at least one propellant, the weight ratio of coloring agent to propellant being in the range of from 5:95 to 95:5, preferably 50:50, particularly preferably 80:20.

15. A method for dyeing keratin fibers, **characterized in that** an agent according to one of claims 1 to 13 is applied from a dispenser, preferably from a spray can according to claim 11 or from a non-aerosol mousse dispenser, as a mousse, the resulting mousse is subsequently distributed onto the keratin fibers, then left on the keratin fibers for a period of from 30 seconds to 60 minutes, preferably from 5 to 45 minutes, and is subsequently washed out of the keratin fibers.

## Revendications

1. Agent cosmétique contenant de l'eau pour la teinture de fibres kératiniques
- ayant une valeur de pH dans la plage de 1,0 à 5,5 et
- contenant au moins un colorant acide à action directe et
- par rapport au poids de l'agent, 6 à 12 % en poids de solvant organique, dont 80 à 100 % en poids sont constitués de carbonate de propylène et 0 à 20 % en poids d'alcool benzylique,
- et au moins un tensioactif éthoxylé non ionique en une quantité totale de 0,1 à 1 % en poids, par rapport au poids de l'agent,
la teneur totale en solvant organique étant de 6 à 12 % en poids, par rapport au poids de l'agent.

2. Agent selon la revendication 1, **caractérisé en ce qu'**il contient au moins un colorant acide à action directe du groupe Acid Yellow 1, Acid Yellow 3, Acid Yellow 9, Acid Yellow 17, Acid Yellow 23, Acid Yellow 36, Acid Yellow 121, Acid Orange 6, Acid Orange 7, Acid Orange 10, Acid Orange 11, Acid Orange 15, Acid Orange 20, Acid Orange 24, Acid Red 14, Acid Red 18, Acid Red 27, Acid Red 33, Acid Red 35, Acid Red 51, Acid Red 52, Acid Red 73, Acid Red 87, Acid Red 95, Acid Red 184, Acid Red 195, Acid Violet 43, Acid Violet 49, Acid Violet 50, Acid Blue 1, Acid Blue 3, Acid Blue 7, Acid Blue 104, Acid Blue 9, Acid Blue 62, Acid Blue 74, Acid Blue 80, Acid Green 3, Acid Green 5, Acid Green 9, Acid Green 22, Acid Green 25, Acid Green 50, Acid Black 1, Acid Black 52, Food Yellow 8, Food Blue 5, D&C Yellow 7, D&C Yellow 8, D&C Orange 4, D&C Green 5, D&C Orange 10, D&C Orange 11, D&C Red 21, D&C Red 27, D&C Red 33, D&C Violet 2 et/ou D&C Brown 1.

3. Agent selon l'une quelconque des revendications 1 ou 2, **caractérisé en ce qu'**il contient au moins l'une des combinaisons suivantes de colorants acides à action directe : Acid Yellow 1/ Acid Orange 7 ; Acid Yellow 3/ Acid Orange 7 ; Acid Yellow 9/ Acid Orange 7 ; Acid Yellow 17/ Acid Orange 7 ; Acid Yellow 23/ Acid Orange 7 ; Acid Yellow 36/ Acid Orange 7 ; Acid Yellow 121/Acid Orange 7 ; Acid Red 14/Acid Orange 7 ; Acid Red 18/ Acid Orange 7 ; Acid Red 27/ Acid Orange 7 ; Acid Red 33/ Acid Orange 7 ; Acid Red 35/ Acid Orange 7 ; Acid Red 51/ Acid Orange 7 ; Acid Red 52/ Acid Orange 7 ; Acid Red 73/ Acid Orange 7 ; Acid Red 87/ Acid Orange 7 ; Acid Red 95/ Acid Orange 7 ; Acid Red 184/ Acid Orange 7 ; Acid Red 195/ Acid Orange 7 ; Acid Violet 43/ Acid Orange 7 ; Acid Violet 49/ Acid Orange 7 ou Acid Violet 50/ Acid Orange 7.

4. Agent selon l'une quelconque des revendications 1 à 3, **caractérisé en ce qu'**il contient un ou plusieurs colorants acides à action directe en une quantité totale de 0,01 à 5,5 % en poids, de préférence de 0,08 à 4,7 % en poids, de manière davantage préférée de 0,2 à 3,4 % en poids et de manière particulièrement préférée de 0,3 à 1,8 % en poids, par rapport au poids de l'agent.

5. Agent selon l'une quelconque des revendications 1 à 4, **caractérisé en ce qu'**il présente un pH de 1,5 à 4,7, de préférence de 1,6 à 3,9, de manière davantage préférée de 1,7 à 3,1 et de manière particulièrement préférée de 1,8 à 2,5.

6. Agent selon l'une quelconque des revendications 1 à 5, **caractérisé en ce qu'**il contient, dans chaque cas par rapport à son poids, de 4,8 à 12 % en poids, de préférence de 8 à 10 % en poids et de manière particulièrement préférée de 8,5 à 9,6 % en poids de carbonate de propylène et de 0 à 2,4 % en poids, de préférence de 0,18 à 2,0 % en poids et de manière particulièrement préférée de 1,0 à 1,8 % en poids d'alcool benzylique.

7. Agent selon l'une quelconque des revendications 1 à 6, **caractérisé en ce qu'**il contient au moins un agent tensioactif cationique en une quantité totale de 0,1 à 2 % en poids, de préférence de 0,2 à 1,5 % en poids, de manière particulièrement préférée de 0,4 à 0,8 % en poids, dans chaque cas par rapport au poids de l'agent.

8. Agent selon la revendication 7, **caractérisé en ce que** l'au moins un agent tensioactif cationique est choisi parmi les chlorures d'alkyltriméthylammonium en C10-C22, en particulier choisis parmi le chlorure de béhényltriméthylammonium, le chlorure de stéaryltriméthylammonium et le chlorure de cétyltriméthylammonium, et le quaternium-52 et ainsi que des mélanges de ces agents tensioactifs.

9. Agent selon l'une quelconque des revendications 1 à 8, **caractérisé en ce qu'**il contient au moins un agent tensioactif non ionique éthoxylé en une quantité totale de 0,2 à 0,8 % en poids, de manière particulièrement préférée de 0,3 à 0,7 % en poids, dans chaque cas par rapport au poids de l'agent.

10. Agent selon l'une quelconque des revendications 1 à 9, **caractérisé en ce que** l'au moins un agent tensioactif éthoxylé non ionique est choisi parmi les produits d'addition d'oxyde d'éthylène avec des alcools gras et des acides gras saturés ou insaturés, linéaires ou ramifiés et avec des esters d'acides gras de glycérol avec 2 à 60 moles d'oxyde d'éthylène par mole d'alcool gras ou d'acide gras ou d'ester d'acide gras de glycérol et ainsi que des mélanges de ceux-ci, en particulier choisis parmi la PEG-40 Hydrogenated Castor Oil et la PEG-60 Hydrogenated Castor Oil ainsi que des mélanges de ceux-ci.

11. Agent selon l'une quelconque des revendications 1 à 10, **caractérisé en ce qu'**il contient au moins un polymère cationique en une quantité totale de 0,01 à 2 % en poids, de préférence de 0,1 à 1,5 % en poids, de manière particulièrement préférée de 0,5 à 1,0 % en poids, dans chaque cas par rapport au poids de l'agent.

12. Agent selon la revendication 11, **caractérisé en ce que** le polymère cationique est choisi dans le groupe constitué de polyquaternium-1, de polyquaternium-2, de polyquaternium-3, de polyquaternium-4, de polyquaternium-5, de polyquaternium-6, de polyquaternium-7, de polyquaternium-8, de polyquaternium-9, de polyquaternium-10, de polyquaternium-11, de polyquaternium-14, de polyquaternium-16, de polyquaternium-17, de polyquaternium-18, de polyquaternium-22, de polyquaternium-24, de polyquaternium-27, de polyquaternium-28, de polyquaternium-32, de polyquaternium-33, de polyquaternium-37, de polyquaternium-39, de polyquaternium-44, de polyquaternium-46, polyquaternium-53, polyquaternium-55, polyquaternium-64, polyquaternium-67, polyquaternium-68, polyquaternium-69 et polyquaternium-86, ainsi que de mélanges de ceux-ci, de préférence choisi parmi les mélanges de polyquaternium-4 et de polyquaternium-11.

13. Agent selon l'une quelconque des revendications 1 à 12, **caractérisé en ce qu'**il contient au moins un polymère non ionique de polyvinylpyrrolidone/acétate de vinyle en une quantité totale de 0,1 à 2,0 % en poids, de préférence de 0,2 à 1,5 % en poids, de manière particulièrement préférée de 0,4 à 1,0 % en poids, dans chaque cas par rapport au poids de l'agent.

14. Produit de teinture des cheveux, **caractérisé en ce qu'**il comprend un récipient aérosol et, conditionné dans celui-ci, un colorant selon l'une des revendications 1 à 13 et au moins un agent gonflant, le rapport pondéral entre le colorant et l'agent gonflant étant dans la plage de 5:95 à 95:5, de préférence 50:50, de manière particulièrement préférée 80:20.

15. Procédé de teinture de fibres kératiniques, **caractérisé en ce qu'**un agent selon l'une quelconque des revendications 1 à 13 est distribué sous forme de mousse à partir d'un distributeur, de préférence à partir d'une bombe aérosol selon la revendication 11 ou à partir d'un distributeur de mousse non-aérosol, puis la mousse ainsi obtenue est distribuée sur les fibres kératiniques, puis repose sur les fibres kératiniques pendant une durée de 30 secondes à 60 minutes, de préférence de 5 à 45 minutes, et est ensuite éliminée des fibres kératiniques par lavage.
